(19) Europäisches Patentamt
European Patent Office
Office européen des brevets

(11) **EP 3 882 625 B1**

(12) **EUROPÄISCHE PATENTSCHRIFT**

(45) Veröffentlichungstag und Bekanntmachung des Hinweises auf die Patenterteilung:
**07.02.2024 Patentblatt 2024/06**

(21) Anmeldenummer: **21160418.6**

(22) Anmeldetag: **03.03.2021**

(51) Internationale Patentklassifikation (IPC):
***G01N 33/00*** *(2006.01)*

(52) Gemeinsame Patentklassifikation (CPC):
**G01N 33/0006**

(54) **VERFAHREN ZUM JUSTIEREN EINES GASVERSORGUNGSSYSTEMS UND GASVERSORGUNGSSYSTEM MIT JUSTIERFUNKTION**

METHOD FOR ADJUSTING A GAS SUPPLY SYSTEM AND GAS SUPPLY SYSTEM WITH ADJUSTMENT FUNCTION

PROCÉDÉ D'AJUSTEMENT D'UN SYSTÈME D'ALIMENTATION EN GAZ ET SYSTÈME D'ALIMENTATION EN GAZ À FONCTION D'AJUSTEMENT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **17.03.2020 DE 102020001756**

(43) Veröffentlichungstag der Anmeldung:
**22.09.2021 Patentblatt 2021/38**

(73) Patentinhaber: **Dräger Safety AG & Co. KGaA 23560 Lübeck (DE)**

(72) Erfinder: **Gnoerrlich, Tim 23558 Lübeck (DE)**

(74) Vertreter: **Guthöhrlein, Gerhard Drägerwerk AG & Co. KGaA Moislinger Allee 53 - 55 23558 Lübeck (DE)**

(56) Entgegenhaltungen:
CN-A- 109 580 887          DE-A1- 4 339 472
DE-A1-102015 012 440       US-A1- 2011 108 418
US-A1- 2011 265 550

**Beschreibung**

Die Erfindung betrifft ein Verfahren zum Justieren eines Gasversorgungssystems und ein Gasversorgungssystem mit Justierfunktion

STAND DER TECHNIK

Elektrochemische und katalytische Sensoren zur Überwachung von toxischen bzw. explosiven Gasen und Dämpfen sind vielfach in Gebrauch. Es ist bekannt, dass Sensoren, insbesondere elektrochemische Sensoren, im Laufe ihrer Lebenszeit ihre Messeigenschaften, insbesondere ihre Sensitivität, also ihre Empfindlichkeit und/oder ihre Ansprechzeit, bis ein Messwert 90 % einer wirklich anliegenden Gaskonzentration erreicht, verändern und daher regelmäßig überprüft, insbesondere kalibriert werden müssen.

Eine Kalibration bzw. Kalibrierung erfolgt in der Regel in einem Zweischrittverfahren, bei dem in einem ersten Schritt ein Nullpunkt durch Beaufschlagung mit sogenanntem "Nullgas", also einem Gas, das keine Beimengungen enthält, bestimmt wird. In einem zweiten Schritt wird die Sensitivität eines jeweiligen Sensors durch Beaufschlagung mit einem Prüfgas, also einem Gas mit einer definierten Konzentration an Beimengungen, ermittelt.

Zum Erzeugen eines Prüfgases, kann ein Gasgenerator genutzt werden, der das Prüfgas in regelmäßigen Abständen produziert, um einen jeweiligen Sensor bspw. automatisch zu überprüfen bzw. zu kalibrieren. Alternativ kann ein Prüfgas mit einer Gasflasche bereitgestellt werden.

Bei einer Kalibrierung ohne Gasgenerator muss ein Betreiber bzw. ein Nutzer, die Kalibrierung manuell vor Ort an einem jeweiligen Sensor mittels einer Gasflasche durchführen, was einerseits aufwändig, andererseits ggf. für den Betreiber in entsprechender Atmosphäre gefährlich sein kann.

Um falsche Interpretationen von durch einen Sensor ermittelten Messwerten zu vermeiden, kann ein jeweiliges Messsystem justiert werden, indem ein Nullpunkt und eine Sensitivität des Sensors in Abhängigkeit einer während einer Überprüfung bzw. einer Kalibrierung erkannten Abweichung zwischen einem aktuellen Zustand und einem Referenzzustand des Sensors angepasst bzw. korrigiert wird.

Wird ein Gasgenerator zur Kalibrierung bzw. Justierung eingesetzt, so unterliegt auch dieser einem Alterungsprozess und generiert über die Lebenszeit bei gleichem Stimulus immer weniger Gas, was von einem automatischen System zur Kalibrierung bzw. Justierung eines Sensors als Abfall eines Antwortverhaltens des Sensors interpretiert wird, sodass es zu Situationen kommen kann, in denen der Sensor unnötig getauscht bzw. falsch fehlerhaft gemeldet wird. Die Empfindlichkeit des Sensors wird entsprechend als zu geringgeschätzt.

US 2011/0265550 A1 beschreibt eine Messapparatur zum Erfassen von kohlenstoffhaltigen Gasen in Luft.

OFFENBARUNG DER ERFINDUNG

Vor dem voranstehend beschriebenen technischen Hintergrund hat der Erfindung die Aufgabe zugrunde gelegen, ein Gasversorgungssystem bereitzustellen, das die voranstehend beschriebenen Nachteile zumindest teilweise nicht aufweist. Insbesondere ist es eine Aufgabe der vorliegenden Erfindung, einen Zeitbereich zwischen zwei Kalibrierungsvorgängen zur Kalibrierung eines Sensors eines Gasmessgeräts zu maximieren.

Voranstehende Aufgabe wird gelöst durch ein Verfahren und ein Gasversorgungssystem mit den Merkmalen der jeweiligen unabhängigen Ansprüche. Weitere Merkmale und Details der Erfindung ergeben sich aus den Unteransprüchen, der Beschreibung und den Zeichnungen. Dabei gelten Merkmale und Details, die im Zusammenhang mit dem erfindungsgemäßen Verfahren beschrieben sind, selbstverständlich auch im Zusammenhang mit dem erfindungsgemäßen Gasversorgungssystem und jeweils umgekehrt, sodass bezüglich der Offenbarung zu den einzelnen Erfindungsaspekten stets wechselseitig Bezug genommen wird beziehungsweise werden kann.

Es wird somit ein Verfahren zum Justieren eines Gasversorgungssystems vorgestellt. Das Verfahren umfasst einen Kalibrierungsschritt zum Kalibrieren eines Gassensors zu einem ersten Kalibrierungszeitpunkt mit einer definierten Konzentration an Prüfgas, wobei während des Kalibrierens ein erstes Kalibrierungsergebnis ermittelt wird, und wobei mindestens ein zu einem dem ersten Kalibrierungszeitpunkt vorangegangenen Zeitpunkt ermittelter Gasversorgungsmesswert ermittelt wird, einen Ermittlungsschritt zum Ermitteln eines Gasversorgungsreferenzwerts, indem der mindestens eine Gasversorgungsmesswert in eine mathematische Beziehung gesetzt wird mit dem ersten Kalibrierungsergebnis und einem weiteren Kalibrierungsergebnis des Gassensors, das zu einem dem ersten Kalibrierungszeitpunkt vorangegangenen Zeitpunkt ermittelt wurde, und einen Justierungsschritt zum Justieren des Gasversorgungssystems anhand des Gasversorgungsreferenzwerts.

Unter einer Kalibrierung bzw. einem Kalibrieren oder einer Kalibration ist im Kontext der vorgestellten Erfindung ein Vorgang zu verstehen, bei dem ein aktueller Zustand eines Gassensors und/oder einer Gasversorgung des vorgestellten Gasmessgeräts relativ zu einem Referenzzustand ermittelt wird.

Unter einer Justierung bzw. einem Justieren ist im Kontext der vorgestellten Erfindung ein Vorgang zu verstehen, bei dem der Gassensor und/oder die Gasversorgung des vorgestellten Gasversorgungssystems in Abhängigkeit eines aktuell ermittelten Zustands des Gassensors und/oder der Gasversorgung angepasst bzw. kontrolliert, d.h. gesteuert oder geregelt werden.

Insbesondere ist unter einer Justierung ein Vorgang zu verstehen, bei dem Ergebnisse einer Kalibrierung zur Korrektur des Gasversorgungssystems bzgl. einer Abweichung zwischen einem aktuellen Zustand und einem Referenzzustand verwendet werden. Beispielsweise kann bei einer Justierung ein jeweiliger Messwert bzw. ein Antwortverhalten eines jeweiligen Gassensors angepasst bzw. korrigiert werden.

Unter einer Recheneinheit ist im Kontext der vorgestellten Erfindung eine programmierbare Prozessoreinheit bzw. Subprozessoreinheit zu verstehen. Eine Recheneinheit kann als Computer, insbesondere als verteiltes Rechensystem ausgestaltet sein und mit einem Speicher, wie bspw. einem Netzwerkspeicher oder einem Feststoffspeicher in kommunikativem Kontakt stehen. Insbesondere kann eine Recheneinheit ein Steuergerät, wie bspw. ein Microcontroller sein.

Unter einer definierten Konzentration an Prüfgas ist im Kontext der vorgestellten Erfindung eine vorgegebene bzw. bekannte Konzentration an Prüfgas zu verstehen, die bspw. händisch bei einem Kalibrierungsvorgang aus einer Prüfgasflasche oder mittels eines Gasgenerators bereitgestellt wird. Dabei kann die definierte Konzentration an Prüfgas um einen Toleranzbetrag schwanken.

Das vorgestellte Verfahren dient insbesondere zum Justieren einer Gasversorgung, insbesondere eines Gasgenerators, die zum Kalibrieren bzw. Justieren eines Sensors verwendet wird. Dazu ist vorgesehen, dass eine Gasversorgung während einer Kalibrierung bzw. Justierung eines jeweiligen Sensors mit justiert wird.

Durch eine justierte Gasversorgung kann eine fehlerhafte Prüfung eines Sensors, bspw. bedingt durch eine falsche Ansteuerung der Gasversorgung, vermieden werden. Entsprechend kann ein Zeitbereich zwischen zwei Kalibrierungsvorgängen eines Sensors unabhängig von einem Alterungszustand der Gasversorgung gewählt und entsprechend maximiert werden.

Das vorgestellte Verfahren basiert darauf, dass ein aktueller bzw. erster Kalibrierungsvorgang eines Sensors genutzt wird, um auf einen Alterungszustand einer Gasversorgung, die den Sensor mit Prüfgas beaufschlagt, zu schließen. Dazu wird während des ersten Kalibrierungsvorgangs, also zu einem ersten Kalibrierungszeitpunkt, ein erstes Kalibrierungsergebnis des Sensors, also eine Sensitivität des Sensors durch eine Beaufschlagung mit Prüfgas, ermittelt.

Zusätzlich zu dem ersten Kalibrierungsergebnis wird mindestens ein Gasversorgungsmesswert zu einem Zeitpunkt ermittelt, der zeitlich vor, insbesondere zeitlich unmittelbar vor, dem ersten Kalibrierungszeitpunkt durch den Sensor erfasst wurde. In Verbindung mit einem weiteren Kalibrierungsergebnis, das vor dem ersten Kalibrierungszeitpunkt ermittelt wurde, kann anhand des Gasversorgungsmesswerts und des ersten Kalibrierungsergebnisses ein Gasversorgungsreferenzwert ermittelt werden. Dies bedeutet, dass eine Veränderung der Sensitivität des Sensors zwischen zwei Kalibrierungsvorgängen mathematisch in Beziehung gesetzt wird mit einem vor dem ersten Kalibrierungsvorgang ermittelten Gasversorgungsmesswert, insbesondere einem letzten Gasversorgungsmesswert vor dem ersten Kalibrierungsvorgang, um den Gasversorgungsreferenzwert zu ermitteln.

Sobald der Gasversorgungsreferenzwert bekannt ist, kann dieser dazu verwendet werden, eine entsprechende Gasversorgung zu justieren, d.h. so einzustellen, dass eine Menge an durch die Gasversorgung zu erzeugendem Prüfgas bzw. eine Aktivierungszeit der Gasversorgung unter Verwendung des Gasversorgungsreferenzwerts ermittelt wird.

Zum Ermitteln des Gasversorgungsmesswerts kann vorgesehen sein, dass ein jeweiliger Sensor regelmäßig, insbesondere zwischen jeweiligen Kalibrierungsvorgängen mit einem Prüfgas beaufschlagt wird.

Durch ein mathematisches in Beziehung setzen einer Veränderung einer Sensitivität des Sensors bzw. einer Alterung des Sensors mit einem Gasversorgungsmesswert kann der quantitative Anteil einer Abweichung des Gasversorgungsmesswerts zu einem Sollwert, der durch die Alterung der Gasversorgung bedingt ist, ermittelt werden. Der quantitative Anteil der Abweichung des Gasversorgungsmesswerts zu dem Sollwert, der durch die Alterung der Gasversorgung bedingt ist kann, sobald dieser bekannt ist, zur Justierung der Gasversorgung verwendet werden, sodass dieser Anteil ausgeglichen oder bei weiteren Kalibrierungsvorgängen berücksichtigt wird.

Weiterhin kann anhand des quantitativen Anteils der Abweichung des Gasversorgungsmesswerts zu einem Sollwert, d.h. der Alterung der Gasversorgung, ein Kriterium bestimmt werden, wann die Gasversorgung ausgewechselt werden muss, weil seine generierte Gasmenge und ein entsprechendes Signal-zu-Rauschverhältnis zu gering sind.

Insbesondere kann mittels des vorgestellten Verfahrens nach einer Justierung eines Gassensors eine Alterung des Gassensors, d.h. ein auf physikalische Eigenschaften des Gassensors begründeter Abfall von Messwerten seit einer vorherigen Kalibrierung bzw. Justierung des Gassensors bestimmt werden. Diese Alterung des Gassensors kann mit einem Ergebnis eines Sensitivitätsabfalls einer Gasversorgung verglichen werden. Dabei entspricht die relative Alterung des Gasgenerators einem Verhältnis eines aktuell ermittelten Referenzwerts und eines alten bzw. vor dem aktuell ermittelten Referenzwert gültigen Referenzwerts. Entsprechend ist der Gasgenerator nicht gealtert, wenn zu verschiedenen Zeitpunkten ermittelte Referenzwerte konstant bzw. gleich sind.

Das erfindungsgemäß vorgesehene mathematische in Beziehung setzen kann ein Ermitteln einer Abfallcharakteristik, wie bspw. einer Steigung einer Anzahl von Gasversorgungsmesswerten umfassen. Entsprechend kann eine ermittelte Abfallcharakteristik bei dem erfindungsgemäßen Justiervorgang korrigiert werden, indem das Gasversorgungssystem zum Ausgleichen der Abfallcharakteristik konfiguriert wird. Dazu kann bspw. ein entsprechender Gasversorgungsreferenzwert an dem Gasversorgungssystem eingestellt und/oder ein Antwortverhalten eines jeweiligen Gassensors angepasst bzw. korrigiert werden.

Es kann vorgesehen sein, dass das Kalibrieren des Gassensors und das Ermitteln des Gasversorgungsreferenzwerts zu einem gegenüber dem ersten Kalibrierungszeitpunkt späteren Zeitpunkt wiederholt werden, wobei dann das erste Kalibrierungsergebnis als weiteres Kalibrierungsergebnis verwendet wird, ein zu dem späteren Zeitpunkt ermitteltes Kalibrierungsergebnis als erstes Kalibrierungsergebnis verwendet wird und mindestens ein zu einem vor dem späteren Zeitpunkt liegenden Zeitpunkt ermittelter Gasversorgungsmesswert als mindestens einer Gasversorgungsmesswert verwendet wird.

Durch eine wiederholte Ausführung jeweiliger Teilschritte des vorgestellten Verfahrens kann ein Alterungsprozess einer jeweiligen Gasversorgung kontinuierlich überwacht und entsprechend korrigiert werden. Daher kann ein Einfluss des Alterungsprozesses der Gasversorgung auf einen Kalibrierungsvorgang minimiert und ein jeweiliger Sensor unabhängig von einem Alterungszustand der Gasversorgung justiert werden.

Es kann weiterhin vorgesehen sein, dass der Gasversorgungsreferenzwert ermittelt wird, indem der mindestens eine Gasversorgungsmesswert multipliziert wird mit einem Verhältnis des weiteren Kalibrierungsergebnisses und des ersten Kalibrierungsergebnisses.

Die Rechenvorschrift (1) zum Ermitteln des Gasversorgungsreferenzwerts hat sich als besonders vorteilhaft erwiesen, wobei folgendes gilt: S1 entspricht einem ersten Kalibrierungsergebnis, S0 einem weiteren Kalibrierungsergebnis, M1 einem Gasversorgungsmesswert und G1 einem Gasversorgungsreferenzwert. Dabei kann M1 zum Beispiel ein Mittelwert bzw. Medianwert einer Anzahl N letzter Gasversorgungsmesswerte vor einem ersten Kalibrierungsvorgang zum Ermitteln des ersten Kalibrierungsergebnisses S1 sein.

$$G1=M1_*(S0/S1)$$

Es kann weiterhin vorgesehen sein, dass der mindestens eine Gasversorgungsmesswert (M1) durch einen der folgenden Ermittlungsschritte ermittelt wird:

  a) Ermitteln eines Integrals einer Sensorantwort des Gassensors (303, 401)
  b) Ermitteln einer Steigung einer Sensorantwort des Gassensors (303, 401),
  c) Ermitteln einer Ansprechzeit einer Sensorantwort des Gassensors (303, 401),
  d) Ermitteln eines Kurvenverlaufs einer Sensorantwort des Gassensors (303, 401).

Es kann weiterhin vorgesehen sein, dass der Gasversorgungsreferenzwert mit einem vorgegebenen Kriterium abgeglichen wird, um zu bestimmen, ob die Gasversorgung ausgewechselt werden muss.

Insbesondere kann vorgesehen sein, dass eine Warnmeldung zum Auswechseln der Gasversorgung ausgegeben wird, wenn eine Differenz zwischen dem Gasversorgungsmesswert und dem Gasversorgungsreferenzwert sich von einem vorgegebenen Schwellenwert unterscheidet.

Da eine Differenz zwischen dem Gasversorgungsmesswert und dem Gasversorgungsreferenzwert einen Alterungszustand der Gasversorgung angibt, kann die Differenz als Maß für eine Auswechslung der Gasversorgung verwendet werden. So kann, sollte bspw. die Differenz größer werden als ein vorgegebener Schwellenwert, davon ausgegangen werden, dass die Gasversorgung alt bzw. verschlissen ist und nichtmehr zum Kalibrieren eines Sensors geeignet ist.

Es kann weiterhin vorgesehen sein, dass ein Wert der Konzentration an Prüfgas, die mittels der Gasversorgung bereitgestellt wird, auf den Gasversorgungsreferenzwert korrigiert wird, d.h. bspw. die Konzentration auf den Gasversorgungsreferenzwert aufsummiert wird, oder die Gasversorgung unter Verwendung des Gasversorgungsreferenzwerts derart gesteuert wird, dass die Konzentration an Prüfgas, die mittels der Gasversorgung bereitgestellt wird, einem vorgegebenen Sollwert entspricht.

Mittels des erfindungsgemäß berechneten Gasversorgungsreferenzwerts kann eine Gasversorgung derart kontrolliert, d.h. gesteuert oder geregelt werden, dass altersbedingte Veränderungen der Gasversorgung ausgeglichen werden.

Zum Justieren des erfindungsgemäß vorgesehenen Gasversorgungssystems kann eine erwartete Prüfgaskurvenform des Gasversorgungssystems kalibriert und justiert werden. Dabei kann bspw. für den Fall, wenn über eine Prüfgaskurvenform bspw. ein Verstopfungszustand eines Sensoreingangs festgestellt wird, die Prüfgaskurvenform über die Zeit jedoch ändert, bei einer Sensorkalibrierung auch diese Referenzkurvenform justiert werden.

Weiterhin kann mittels des vorgestellten Verfahrens ein Gasversorgungssignal, bspw. ein sogenanntes "Time to Peak des Prüfgasimpulses" zum Bestimmen einer Ansprechzeit, bspw. einer sogenannten "T90-Zeit" eines jeweiligen Sensors kalibriert und/oder justiert werden.

In einem zweiten Aspekt betrifft die vorgestellte Erfindung ein Gasversorgungssystem mit Justierungsfunktion. Das Gasversorgungssystem umfasst eine Schnittstelle zu einem Gassensor, eine Gasversorgung und eine Recheneinheit. Die Recheneinheit ist dazu konfiguriert, ein erstes Kalibrierungsergebnis zu einem ersten Kalibrierungszeitpunkt zum Kalibrieren des Gassensors mit einer definierten Konzentration an Prüfgas zu ermitteln und mindestens einen Gasversorgungsmesswert zu einem dem ersten Kalibrierungszeitpunkt vorangegangenen Zeitpunkt zu ermitteln. Weiterhin ist die Recheneinheit dazu konfiguriert, einen Gasversorgungsreferenzwert zu ermitteln, indem der mindestens eine Gasversorgungsmesswert in eine mathematische Beziehung gesetzt wird mit dem ersten Kalibrierungsergebnis und einem zu einem dem ersten Zeitpunkt vorangegangenen Zeitpunkt ermittelten weiteren Kalibrierungsergebnis des Gassensors, und das Gasversorgungssystems anhand des Gasversorgungsreferenzwerts zu justieren.

Das vorgestellte Verfahren dient insbesondere zum Betrieb des vorgestellten Gasversorgungssystems.

Das vorgestellte Gasversorgungssystem umfasst eine Schnittstelle zu einem Gassensor, sodass die Recheneinheit des Gasversorgungssystems mit dem Gassensor in kommunikativem Kontakt steht und bspw. durch den Gassensor ermittelte Messwerte empfangen kann. Dabei kann die Schnittstelle drahtgebunden oder drahtlos ausgestaltet sein. Entsprechend kann der Gassensor als integraler Bestandteil des Gasversorgungssystems ausgestaltet sein oder als externes Modul lediglich über die Schnittstelle mit dem Gasversorgungssystem verbunden sein. Dies bedeutet, dass das Gasversorgungssystem dazu konfiguriert sein kann, sich selbst zu justieren oder einen externen Gassensor zu justieren.

Das Gasversorgungssystem kann eine Gasflasche mit einem steuerbaren bzw. regelbaren Ventil zum Ablassen von Prüfgas oder einen Gasgenerator zum Erzeugen von Prüfgas umfassen.

BEVORZUGTE AUSFÜHRUNGSBEISPIELE

Weitere, die Erfindung verbessernde Maßnahmen ergeben sich aus der nachfolgenden Beschreibung zu einigen Ausführungsbeispielen der Erfindung, welche in den Figuren dargestellt sind. Es zeigen jeweils schematisch:

Figur 1     einen schematischen Ablauf einer möglichen Ausgestaltung des vorgestellten Verfahrens,

Figur 2     ein Diagramm mit Messwerten zur Durchführung des vorgestellten Verfahrens,

Figur 3     eine mögliche Ausgestaltung des vorgestellten Gasversorgungssystems mit einem integralen Gassensor,

Figur 4     eine mögliche Ausgestaltung des vorgestellten Gasversorgungssystems mit einem externen Gassensor.

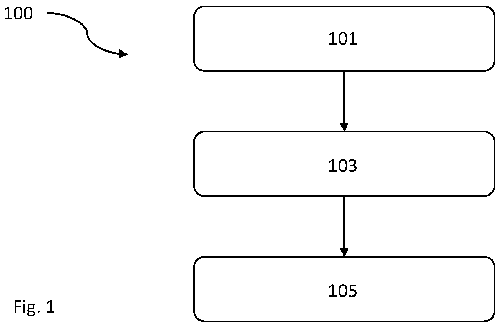

Elemente mit gleicher Funktion und Wirkungsweise sind in den Fig. 1 bis 3 jeweils mit denselben Bezugszeichen versehen.

In Fig. 1 ist ein Verfahren 100 dargestellt. Das Verfahren 100 umfasst einen Kalibrierungsschritt 101 zum Kalibrieren eines Gassensors zu einem ersten Kalibrierungszeitpunkt mit einer definierten Konzentration an Prüfgas, wobei während des Kalibrierens ein erstes Kalibrierungsergebnis ermittelt wird, und wobei mindestens ein zu einem dem ersten Kalibrierungszeitpunkt vorangegangenen Zeitpunkt ermittelter Gasversorgungsmesswert ermittelt wird, einen Ermittlungsschritt 103 zum Ermitteln eines Gasversorgungsreferenzwerts, indem der mindestens eine Gasversorgungsmesswert in eine mathematische Beziehung gesetzt wird mit dem ersten Kalibrierungsergebnis und einem weiteren Kalibrierungsergebnis des Gassensors, das zu einem dem ersten Kalibrierungszeitpunkt vorangegangenen Zeitpunkt ermittelt wurde, und einen Justierungsschritt 105 zum Justieren des Gasversorgungssystems anhand des Gasversorgungsreferenzwerts.

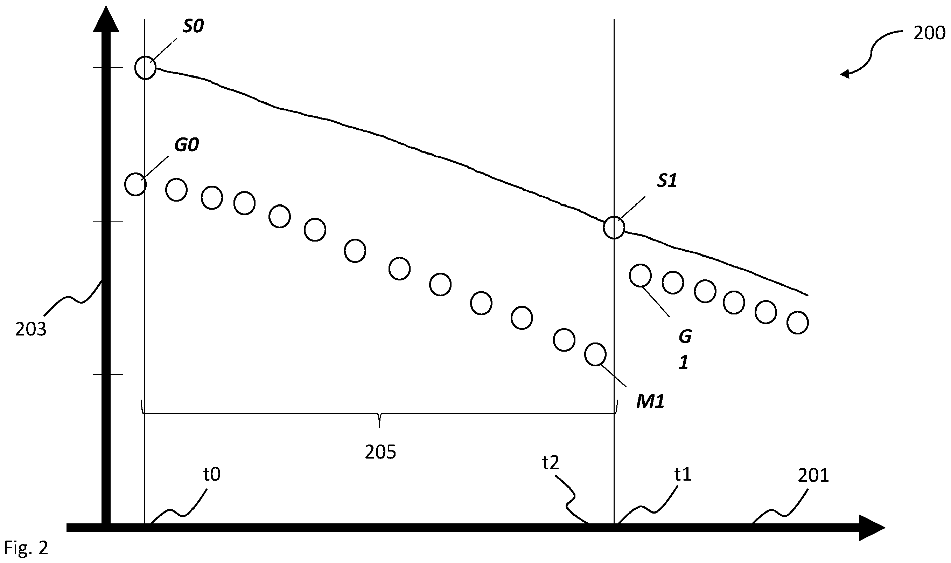

In Fig. 2 ist ein Diagramm 200 dargestellt. Das Diagramm 200 erstreckt sich auf seiner Abszisse 201 über die Zeit und auf seiner Ordinate 203 über eine Sensorsensitivität in $\mu$A/ppm.

Zu einem ersten Kalibrierungszeitpunkt t1 wird ein Gassensor eines Gasversorgungssystems durch bspw. einen Betreiber mit einer Prüfgasflasche, die eine definierte Konzentration an Zielgas bzw. Prüfgas enthält, kalibriert.

Die aus der Prüfgasflasche ausgestoßenen Prüfpartikel wandern zu dem Gassensor, sodass der Gassensor einen Gasversorgungsmesswert mit einer Sensorempfindlichkeit von bspw. 1,5 $\mu$A/ppm misst, die einem ersten Kalibrierungsergebnis S1 entspricht.

Während des ersten Kalibrierungszeitpunkts t1 ist an dem Gasversorgungssystem ein Grundgasversorgungsreferenzwert G0 von bspw. 2000 ppm*s eingestellt, der zu einem Zeitpunkt t0 vor dem ersten Kalibrierungszeitpunkt t1 ermittelt bzw. vorgegebenen wurde. Über die Nutzungszeit des Gassensors und des Gasgenerators ergeben sich abfallende Gasgeneratorsignale 205, sodass ein Gasversorgungsmesswert (M1), der zu einem dem ersten Kalibrierungszeitpunkt (t1) vorangegangenen Zeitpunkt (t2) ermittelt wird, bspw. 1000 ppm*s entspricht.

Ausgehend von dem Gasversorgungsmesswert (M1) würde das Gasversorgungssystem dem Gassensor eine Sensitivität von M1/G0 = 1 $\mu$A /ppm zuordnen, da der Abfall der Gasgeneratorsignale 205 vollständig dem Gassensor zugeordnet würde.

Tatsächlich ist aber auch der Gasgenerator gealtert, sodass eine Betreiberkalibrierung zum ersten Kalibrierungszeitpunkt t1 eine "wahre" Sensorsensitivitat von S1 = 1,5 $\mu$A/ppm ergibt.

Dies bedeutet, dass der Gassensor und der Gasgenerator im gleichen Verhältnis gealtert sind. Ausgehend von dieser Erkenntnis kann ein neuer Gasversorgungsreferenzwert G1 mittels der Berechnungsvorschrift (1) ermittelt werden, wobei S1 einem ersten Kalibrierungsergebnis, S0 einem weiteren Kalibrierungsergebnis, M1 einem Gasversorgungsmesswert und G1 einem Gasversorgungsreferenzwert entspricht. Dabei kann M1 ein Mittelwert bzw. Medianwert einer Anzahl N letzter Gasgeneratorsignale vor einem ersten Kalibrierungsvorgang zum Ermitteln des ersten Kalibrierungsergebnisses S1 sein.

$$G1=M1*(S0/S1) \qquad (1)$$

Das Verfahren 100 kann bspw. mit Sensorrohwerten, von bspw. Strömen eines elektrochemischen Sensors oder mit bereits berechneten Konzentrationswerten eines Sensors genutzt werden.

Zur Bestimmung der Sensitivität des Gassensors während einer Kalibrierung kann eine "Dauerbeaufschlagung" mit Gas durchgeführt werden. Anschließend wird ein stabiler Messwert abgewartet und die Sensitivität bestimmt. Dazu kann bspw. ein Integral einer Sensorantwort des Gassensors auf eine Beaufschlagung mit einem Prüfgas, ein Hub einer Sensorantwort des Gassensors auf eine Beaufschlagung mit einem Prüfgas oder eine Zeit bis zum Peak der Sensorantwort des Gassensors auf eine Beaufschlagung mit einem Prüfgas verwendet werden.

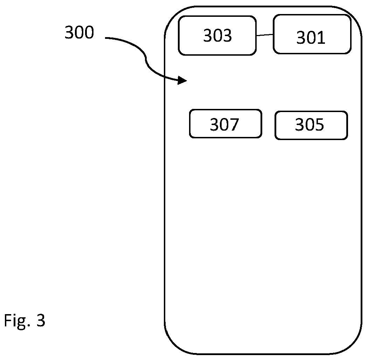

In Fig. 3 ist ein Gasversorgungssystem 300 dargestellt. Das Gasversorgungssystem 300 umfasst eine Schnittstelle 301 zu einem Gassensor, einen Gassensor 303, eine Gasversorgung 305 und eine Recheneinheit 307. Die Recheneinheit ist dazu konfiguriert ein erstes Kalibrierungsergebnis zu einem ersten Kalibrierungszeitpunkt zum Kalibrieren des Gassensors mit einer definierten Konzentration an Prüfgas zu ermitteln.

Weiterhin ist die Recheneinheit 307 dazu konfiguriert, mindestens einen Gasversorgungsmesswert, zu einem dem ersten Kalibrierungszeitpunkt vorangegangenen Zeitpunkt zu ermitteln.

Weiterhin ist die Recheneinheit 307 dazu konfiguriert, einen Gasversorgungsreferenzwerts zu ermitteln, indem der mindestens eine Gasversorgungsmesswert in eine mathematische Beziehung gesetzt wird mit dem ersten Kalibrierungsergebnis und einem zu einem dem ersten Kalibrierungszeitpunkt vorangegangenen Zeitpunkt ermittelten weiteren Kalibrierungsergebnis des Gassensors.

Weiterhin ist die Recheneinheit 307 dazu konfiguriert, das Gasversorgungssystem 300 anhand des Gasversorgungsreferenzwerts zu justieren.

In dem in Fig. 3 gezeigten Beispiel ist der Gassensor 303 über eine elektrische Verbindung, wie bspw. ein Kabel oder eine Lötverbindung mit der Schnittstelle 301 kommunikativ verbunden. Entsprechend übermittelt der Gassensor 303 Messwerte über die Schnittstelle 301 and die Recheneinheit 307.

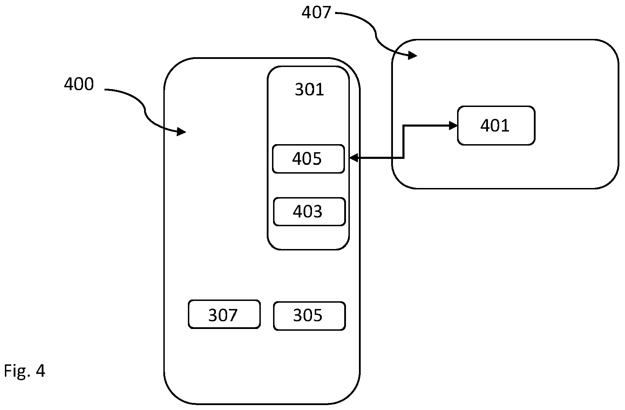

In Fig. 4 ist ein Gasversorgungssystem 400 dargestellt. Das Gasversorgungssystem 400 gleicht dem Gasversorgungssystem 300 gemäß Fig. 3 mit der Ausnahme, dass die Schnittstelle 301 über eine Drahtlosschnittstelle mit einem externen Gassensor 401 in bspw. einem Gasmessgerät 407 verbunden ist. Entsprechend bildet das Gasversorgungssystem 400 ein portables Modul zum Testen verschiedener Gassensoren.

Dabei kann die Schnittstelle 301 ein erstes Kommunikationsmodul 403 zur drahtlosen Kommunikation über bspw. WLAN, Bluetooth, Nearfield Communication, Zigbee oder ein Mobilfunksignal, wie bspw. 5G, und ein zweites Kommunikationsmodul 405 zum Anschluss eines Kabels, wie bspw. einen COM-Port oder einen USB-Port umfassen.

Optional kann das Gasversorgungssystem 400 eine Ausgabeeinheit 409, wie bspw. eine LED, einen Lautsprecher und/oder ein Display umfassen, um Warnmeldungen, wie bspw. einen Hinweis zum Austauschen des Gassensors 401 ausgegeben werden.

## BEZUGSZEICHENLISTE

| | |
|---|---|
| 100 | Verfahren |
| 101 | Kalibrierungsschritt |
| 103 | Ermittlungsschritt |
| 105 | Justierungsschritt |
| 200 | Diagramm |
| 201 | Abszisse |
| 203 | Ordinate |
| 205 | Gasgeneratorsignale |
| t1 | erster Kalibrierungszeitpunkt |
| S1 | erstes Kalibrierungsergebnis |
| M1 | Gasversorgungsmesswert |
| t2 | vorangegangener Zeitpunkt |
| G0 | Gasversorgungsreferenzwert |
| G1 | neuer Gasversorgungsreferenzwert |
| 300 | Gasversorgungssystem |
| 301 | Schnittstelle |
| 303 | Gassensor |
| 305 | Gasversorgung |
| 307 | Recheneinheit |
| 400 | Gasversorgungssystem |
| 401 | externer Gassensor |
| 403 | erstes Kommunikationsmodul |
| 405 | zweites Kommunikationsmodul |
| 407 | Gasmessgerät |
| 409 | Ausgabeeinheit |

**Patentansprüche**

1. Verfahren (100) zum Justieren eines Gasversorgungssystems (300, 400), wobei das Verfahren (100) die folgenden Schritte umfasst:

    - Kalibrieren (101) eines Gassensors (303, 401) zu einem ersten Kalibrierungszeitpunkt (t1) mit einer definierten Konzentration an Prüfgas, wobei während des Kalibrierens ein erstes Kalibrierungsergebnis (S1) ermittelt wird, und **dadurch gekennzeichnet dass** mindestens ein Gasversorgungsmesswert (M1) zu einem dem ersten Kalibrierungszeitpunkt (t1) vorangegangenen Gasversorgungsmesswert-Zeitpunkt (t2) ermittelt wird,

- Ermitteln (103) eines Gasversorgungsreferenzwerts (G1), indem der mindestens eine Gasversorgungsmesswert (M1) in eine mathematische Beziehung gesetzt wird mit dem ersten Kalibrierungsergebnis (S1) und einem Kalibrierungsergebnis (S0) des Gassensors (303, 401), das zu einem dem ersten Kalibrierungszeitpunkt (t1) vorangegangenen Zeitpunkt (t0) ermittelt wurde,

- Justieren (105) des Gasversorgungssystems anhand des Gasversorgungsreferenzwerts (G1), wobei der dem ersten Kalibrierungszeitpunkt (t1) vorangegangene Zeitpunkt (t0), der Gasversorgungsmesswert-Zeitpunkt (t2) und der erste Kalibrierungszeitpunkt (t1) zeitlich aufeinanderfolgen.

2. Verfahren (100) nach Anspruch 1,
   **dadurch gekennzeichnet, dass**
   das Kalibrieren des Gassensors (303, 401) und das Ermitteln des Gasversorgungsreferenzwerts zu einem gegenüber dem ersten Kalibrierungszeitpunkt (t1) späteren Zeitpunkt wiederholt werden, wobei dann das erste Kalibrierungsergebnis (S1) als weiteres Kalibrierungsergebnis verwendet wird, ein zu dem späteren Zeitpunkt ermitteltes Kalibrierungsergebnis als erstes Kalibrierungsergebnis verwendet wird und mindestens ein zu einem vor dem späteren Zeitpunkt liegenden Zeitpunkt ermittelter Gasversorgungsmesswert als mindestens einer Gasversorgungsmesswert (M1) verwendet wird.

3. Verfahren (100) nach Anspruch 1 oder 2,
   **dadurch gekennzeichnet, dass**
   der Gasversorgungsreferenzwert (G1) ermittelt wird, indem der mindestens eine Gasversorgungsmesswert (M1) multipliziert wird mit einem Verhältnis des weiteren Kalibrierungsergebnisses (S0) und des ersten Kalibrierungsergebnisses (S1).

4. Verfahren (100) nach einem der voranstehenden Ansprüche,
   **dadurch gekennzeichnet, dass** der mindestens eine Gasversorgungsmesswert (M1) durch einen der folgenden Ermittlungsschritte ermittelt wird:

   - Ermitteln eines Integrals einer Sensorantwort des Gassensors (303, 401),
   - Ermitteln einer Steigung einer Sensorantwort des Gassensors (303, 401),
   - Ermitteln einer Ansprechzeit einer Sensorantwort des Gassensors (303, 401),
   - Ermitteln eines Kurvenverlaufs einer Sensorantwort des Gassensors (303, 401).

5. Verfahren (100) nach einem der vorangehenden Ansprüche,
   **dadurch gekennzeichnet, dass**
   in Abhängigkeit eines Abgleichs des Gasversorgungsreferenzwerts (G1) mit einem vorgegebenen Kriterium eine Warnmeldung ausgegeben wird.

6. Verfahren (100) nach Anspruch 5,
   **dadurch gekennzeichnet, dass**
   eine Warnmeldung zum Auswechseln einer Gasversorgung (305) des Gasversorgungssystems (300, 400) ausgegeben wird, wenn eine Differenz zwischen dem Gasversorgungsmesswert (M1) und dem Gasversorgungsreferenzwert (G1) sich von einem vorgegebenen Schwellenwert unterscheidet.

7. Verfahren (100) nach einem der vorangehenden Ansprüche,
   **dadurch gekennzeichnet, dass**
   ein Wert der Konzentration an Prüfgas, die mittels der Gasversorgung (305) bereitgestellt wird, auf den Gasversorgungsreferenzwert korrigiert wird oder die Gasversorgung (305) unter Verwendung des Gasversorgungsreferenzwerts derart gesteuert wird, dass die Konzentration an Prüfgas, die mittels der Gasversorgung (305) bereitgestellt wird, einem vorgegebenen Sollwert entspricht.

8. Gasversorgungssystem (300, 400) mit Justierungsfunktion,

   wobei das Gasversorgungssystem (300, 400) umfasst:

   - eine Schnittstelle (301) zu einem Gassensor (303),
   - eine Gasversorgung (305),
   - eine Recheneinheit (307),

   wobei die Recheneinheit (307) dazu konfiguriert ist,
   ein erstes Kalibrierungsergebnis (S1) zu einem ersten Kalibrierungszeitpunkt (t1) zum Kalibrieren eines mit der Schnittstelle verbundenen Gassensors (303) mit einer definierten Konzentration an Prüfgas zu ermitteln, und **dadurch gekennzeichnet dass** die Recheneinheit dazu konfiguriert ist,
   mindestens einen Gasversorgungsmesswert (M1), zu einem dem ersten Kalibrierungszeitpunkt (t1) vorangegangenen Gasversorgungsmesswert-Zeitpunkt (t2) zu ermitteln, und
   einen Gasversorgungsreferenzwert (G1) zu ermitteln, indem der mindestens eine Gasversorgungsmesswert (M1) in eine mathematische Beziehung gesetzt wird mit dem ersten Kalibrierungsergebnis (S1) und einem zu einem dem ersten Kalibrierungszeitpunkt (t1) vorangegangenen Zeitpunkt (t0) ermittelten weiteren Kalibrierungsergebnis (S0) des Gassensors (303), und

das Gasversorgungssystem (300, 400) anhand des Gasversorgungsreferenzwerts (G1) zu justieren,

wobei der dem ersten Kalibrierungszeitpunkt (t1) vorangegangene Zeitpunkt (t0), der Gasversorgungsmesswert-Zeitpunkt (t2) und der erste Kalibrierungszeitpunkt (t1) zeitlich aufeinanderfolgen.

9. Gasversorgungssystem (300, 400) nach Anspruch 8,
   **dadurch gekennzeichnet, dass**
   die Recheneinheit (307) zur Durchführung eines Verfahrens (100) nach einem der Ansprüche 2 bis 7 konfiguriert ist.

10. Gasversorgungssystem (300, 400) nach einem der vorangehenden Ansprüche, **dadurch gekennzeichnet, dass**
    die Gasversorgung (305) einen Gasgenerator oder eine Gasflasche aufweist.

**Claims**

1. Method (100) for adjusting a gas supply system (300, 400), wherein the method (100) comprises the following steps of:

   - calibrating (101) a gas sensor (303, 401) at a first calibration time (t1) with a defined concentration of test gas, wherein a first calibration result (S1) is determined during calibration, and
   **characterized in that**
   at least one gas supply measured value (M1) is determined at a gas supply measured value time (t2) preceding the first calibration time (t1),
   - determining (103) a gas supply reference value (G1) by mathematically relating the at least one gas supply measured value (M1) to the first calibration result (S1) and a calibration result (S0) of the gas sensor (303, 401) that was determined at a time (t0) preceding the first calibration time (t1),
   - adjusting (105) the gas supply system on the basis of the gas supply reference value (G1), wherein the time (t0) preceding the first calibration time (t1), the gas supply measured value time (t2) and the first calibration time (t1) succeed one another in terms of time.

2. Method (100) according to Claim 1,
   **characterized in that**
   the calibration of the gas sensor (303, 401) and the determination of the gas supply reference value are repeated at a time later than the first calibration time (t1), wherein the first calibration result (S1) is then used as a further calibration result, a calibration result determined at the later time is used as a first calibration result and at least one gas supply measured value determined at a time before the later time is used as at least one gas supply measured value (M1).

3. Method (100) according to Claim 1 or 2,
   **characterized in that**
   the gas supply reference value (G1) is determined by multiplying the at least one gas supply measured value (M1) by a ratio of the further calibration result (S0) and the first calibration result (S1).

4. Method (100) according to one of the preceding claims,
   **characterized in that** the at least one gas supply measured value (M1) is determined by means of one of the following determination steps:

   - determining an integral of a sensor response of the gas sensor (303, 401),
   - determining a gradient of a sensor response of the gas sensor (303, 401),
   - determining a response time of a sensor response of the gas sensor (303, 401),
   - determining a curve progression of a sensor response of the gas sensor (303, 401).

5. Method (100) according to one of the preceding claims,
   **characterized in that**
   a warning message is output on the basis of a comparison of the gas supply reference value (G1) with a predefined criterion.

6. Method (100) according to Claim 5,
   **characterized in that**
   a warning message to replace a gas supply (305) of the gas supply system (300, 400) is output if a difference between the gas supply measured value (M1) and the gas supply reference value (G1) differs from a predefined threshold value.

7. Method (100) according to one of the preceding claims,
   **characterized in that**
   a value of the concentration of test gas provided by means of the gas supply (305) is corrected to the gas supply reference value, or the gas supply (305) is controlled using the gas supply reference value in such a manner that the concentration of test gas provided by means of the gas supply (305) corresponds to a predefined target value.

8. Gas supply system (300, 400) with an adjustment function,

wherein the gas supply system (300, 400) comprises:

- an interface (301) to a gas sensor (303),
- a gas supply (305),
- a computing unit (307),

wherein the computing unit (307) is configured to determine a first calibration result (S1) at a first calibration time (t1) for calibrating a gas sensor (303) connected to the interface with a defined concentration of test gas, and **characterized in that** the computing unit is configured to determine at least one gas supply measured value (M1) at a gas supply measured value time (t2) preceding the first calibration time (t1), and to determine a gas supply reference value (G1) by mathematically relating the at least one gas supply measured value (M1) to the first calibration result (S1) and a further calibration result (S0) of the gas sensor (303) determined at a time (t0) preceding the first calibration time (t1), and

to adjust the gas supply system (300, 400) on the basis of the gas supply reference value (G1), wherein the time (t0) preceding the first calibration time (t1), the gas supply measured value time (t2) and the first calibration time (t1) succeed one another in terms of time.

9. Gas supply system (300, 400) according to Claim 8, **characterized in that** the computing unit (307) is configured to carry out a method (100) according to one of Claims 2 to 7.

10. Gas supply system (300, 400) according to one of the preceding claims, **characterized in that** the gas supply (305) has a gas generator or a gas cylinder.

**Revendications**

1. Procédé (100) d'ajustement d'un système d'alimentation en gaz (300, 400), le procédé (100) comprenant les étapes ci-dessous consistant à :

- étalonner (101 un capteur de gaz (303, 401) à un premier instant d'étalonnage (t1) avec une concentration définie de gaz d'essai, un premier résultat d'étalonnage (S1) étant déterminé pendant l'étalonnage, et **caractérisé en ce que** au moins une valeur de mesure d'alimentation en gaz (M1 ) est déterminée à un instant de mesure d'alimentation en gaz (t2) précédant le premier instant d'étalonnage (t1),
- déterminer (103) une valeur de référence d'alimentation en gaz (G1) en mettant la au moins une valeur de mesure d'alimentation en gaz (M1) en relation mathématique avec le premier résultat d'étalonnage (S1) et avec un résultat d'étalonnage (S0) du capteur de gaz (303, 401) ayant été déterminé à un instant (t0) précédant le premier instant d'étalonnage (t1),
- ajuster (105) le système d'alimentation en gaz à l'aide de la valeur de référence d'alimentation en gaz (G1), où l'instant (t0) précédant le premier instant d'étalonnage (t1), l'instant de mesure d'alimentation en gaz (t2) et le premier instant d'étalonnage (t1) se succèdent dans le temps.

2. Procédé (100) selon la revendication 1, **caractérisé en ce que** les étapes d'étalonnage du capteur de gaz (303, 401) et de détermination de la valeur de référence d'alimentation en gaz à un instant ultérieur par rapport au premier instant de calibrage (t1) sont répétées, le premier résultat d'étalonnage (S1) étant ensuite utilisé comme résultat d'étalonnage supplémentaire, un résultat d'étalonnage déterminé audit instant ultérieur étant utilisé comme premier résultat d'étalonnage et au moins une valeur de mesure d'alimentation en gaz déterminée à un instant situé avant ledit instant ultérieur étant utilisée comme au moins une valeur de mesure d'alimentation en gaz (M1).

3. Procédé (100) selon la revendication 1 ou 2, **caractérisé en ce que** la valeur de référence d'alimentation en gaz (G1) est déterminée en multipliant la au moins une valeur de mesure d'alimentation en gaz (M1) par un rapport entre le résultat d'étalonnage supplémentaire (S0) et le premier résultat d'étalonnage (S1).

4. Procédé (100) selon l'une quelconque des revendications précédentes, **caractérisé en ce que** la au moins une valeur de mesure d'alimentation en gaz (M1) est déterminée grâce à l'une des étapes de détermination ci-dessous consistant à :

- déterminer une intégrale d'une réponse de détection du capteur de gaz (303, 401),
- déterminer une pente d'une réponse de détection du capteur de gaz (303, 401),
- déterminer un temps de réponse d'une réponse de détection du capteur de gaz (303, 401),
- déterminer une courbe d'une réponse de détection du capteur de gaz (303, 401).

5. Procédé (100) selon l'une quelconque des revendications précédentes, **caractérisé en ce que** en fonction d'une comparaison de la valeur de référence d'alimentation en gaz (G1) avec un critère prédéfini, un message d'avertissement est émis.

**6.** Procédé (100) selon la revendication 5, **caractérisé en ce que** un message d'avertissement de remplacement d'une alimentation en gaz (305) du système d'alimentation en gaz (300, 400) est émis lorsqu'une différence entre la valeur de mesure d'alimentation en gaz (M1) et la valeur de référence d'alimentation en gaz (G1) diffère d'un seuil prédéfini.

**7.** Procédé (100) selon l'une quelconque des revendications précédentes, **caractérisé en ce que** une valeur de la concentration de gaz d'essai fournie au moyen de l'alimentation en gaz (305) est corrigée à la valeur de référence d'alimentation en gaz, ou bien l'alimentation en gaz (305) est commandée, en utilisant la valeur de référence d'alimentation en gaz, de telle manière que la concentration de gaz d'essai fournie au moyen de l'alimentation en gaz (305) corresponde à une valeur de consigne prédéfinie.

**8.** Système d'alimentation en gaz (300, 400) avec fonction d'ajustement,

le système d'alimentation en gaz (300, 400) comprenant :

- une interface (301) avec un capteur de gaz (303),
- une alimentation en gaz (305),
- une unité de calcul (307),

l'unité de calcul (307) éatnt configurée pour déterminer un premier résultat d'étalonnage (S1) à un premier instant d'étalonnage (t1) afin d'étalonner un capteur de gaz (303) relié à l'interface avec une concentration définie de gaz d'essai,
et **caractérisé en ce que** l'unité de calcul est configurée pour
déterminer au moins une valeur de mesure d'alimentation en gaz (M1) à un instant de mesure d'alimentation en gaz (t2) précédant le premier instant d'étalonnage (t1), et déterminer une valeur de référence d'alimentation en gaz (G1) en mettant la au moins une valeur de mesure d'alimentation en gaz (M1) en relation mathématique avec le premier résultat d'étalonnage (S1) et avec un autre résultat d'étalonnage (S0) du capteur de gaz (303) déterminé à un instant (t0) précédant le premier instant d'étalonnage (t1), et
ajuster le système d'alimentation en gaz (300, 400) à l'aide de la valeur de référence d'alimentation en gaz (G1),
où l'instant (t0) précédant le premier instant d'étalonnage (t1), l'instant de mesure d'alimentation en gaz (t2) et le premier instant d'étalonnage (t1) se succèdent dans le temps.

**9.** Système d'alimentation en gaz (300, 400) selon la revendication 8, **caractérisé en ce que** l'unité de calcul (307) est configurée pour mettre en oeuvre un procédé (100) selon l'une quelconque des revendications 2 à 7.

**10.** Système d'alimentation en gaz (300, 400) selon l'une quelconque des revendications précédentes, **caractérisé en ce que** l'alimentation en gaz (305) présente un générateur de gaz ou une bouteille de gaz.

100

101

103

105

Fig. 1

Fig. 2

Fig. 3

Fig. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20110265550 A1